Europäisches Patentamt

European Patent Office

Office européen des brevets

(11) Publication number: **0 438 596 A1**

## EUROPEAN PATENT APPLICATION
## published in accordance with Art. 158(3) EPC

(21) Application number: 90910924.1

(22) Date of filing: 13.07.90

(86) International application number:
PCT/JP90/00912

(87) International publication number:
WO 91/00734 (24.01.91 91/03)

(51) Int. Cl.⁵: **A61K 35/60**

(30) Priority: 13.07.89 JP 181538/89

(43) Date of publication of application:
31.07.91 Bulletin 91/31

(84) Designated Contracting States:
CH DE ES FR GB IT LI

(71) Applicant: **NIPPON HYPOX LABORATORIES INCORPORATED**
**1759, Matsugaya Hachioji-shi**
**Tokyo 192-03(JP)**

(72) Inventor: **SATOH, Toshio**
**57-3, Nagao Jorokucho**
**Tokushima-shi Tokushima-ken 771-42(JP)**

(74) Representative: **Hansen, Bernd, Dr. Dipl.-Chem. et al**
**Hoffmann, Eitle & Partner Patent- und Rechtsanwälte Arabellastrasse 4**
**W-8000 München 81(DE)**

(54) **SOLID AND AQUEOUS TREATED PRODUCTS OF LAMPREY.**

(57) Solid and aqueous treated products of lamprey as the raw material for food, drink and drugs, prepared by treating lamprey in a specified manner to recover efficiently the components exhibiting an immediate action of preventing and in particular treating diseases of the cardiovascular system, thus solving the problem of the heretofore used fatty oil of lamprey that takes a relatively long period of time to exhibit its effect.

EP 0 438 596 A1

## TECHNICAL FIELD

The present invention relates to a processed solid material and a processed aqueous material of lamprey used as raw materials for production of foods, drinks, medicines, etc.

## BACKGROUND ART

So-called Cyclostomes, such as river lamprey (*Lampetra appendix*), Japanese river lamprey (*Lampetra japonica*), south river lamprey (*Lampetra gagei*), sea lamprey (*Petromyzon marinus*), sand lamprey (*Lampetra reissneri*), pacific lamprey (*Lampetra japonicus*), hag fishes (*Eptatretus burgei*), etc., (hereinafter generically referred to as "lamprey") have been used for a long time as medicinal food which is efficacious for nutrition/ tonicization, prevention/therapy of night blindness (nyctalopia) and circulatory disease, etc.

Heretofore, the medicinal efficacy of lamprey for prevention/ therapy of circulatory disease has been regarded as caused by the vascular wall strengthening action of vitamin E relatively abundantly contained in the oil component of lamprey and the ischemic myocardium protecting action of EPA (eicosapentaenoic acid) and DHA (docosahexaenoic acid). Therefore, in the case of use of lamprey for the purpose of prevention/therapy of circulatory disease, lamprey has been directly cooked and eaten or has been used as processed food containing fatty oil as a main component obtained from lamprey.

In the case where an attempt to strengthen the vascular walls by taking various components contained in the fatty oil of lamprey for the purpose of prevention/therapy of circulatory disease is made, however, a relatively long period of treatment will be required before it shows noticeable efficacy. In particular, in the case where lamprey is used for the purpose of therapy of circulatory disease, a problem arises in that the efficacy thereof is insufficient.

An object of the present invention is therefore to provide a processed solid material and a processed aqueous material of lamprey in which components of lamprey having an immediate effect on prevention/therapy of circulatory disease, in particular, on the therapy of the disease, can be efficiently taken in.

## DISCLOSURE OF THE INVENTION

As a result of various examinations to attain the aforementioned object, the present inventors have found that a material prepared by applying a specific treatment to lamprey has a peripheral vasoconstricting action, a peripheral vasodilating action, a myocardial constractile force increasing action, a spontaneous motion stimulating action, etc. and has efficacy for prevention/therapy of circulatory disease and, in particular, has an immediate effect on therapy of the disease. The present invention has been made based on these findings.

That is, the processed solid material of lamprey according to the present invention is characterized in that it comprises a residue obtained by at least once performing an oil and fat extracting operation on lamprey or its ground material, or comprises a dried material of the residue.

Further, the processed aqueous material of lamprey according to the present invention is classified into three groups: one (hereinafter called "processed aqueous material I") which is characterized in that it comprises an extract solution prepared by performing a solid-liquid extracting operation using water as an extractant on the above mentioned processed solid material of lamprey as a raw material; another one (hereinafter called "processed aqueous material II") which is characterized in that it is produced by applying a heat treatment at a liquid temperature of 50 to 100°C for a period of 1 minute to 2 hours to the processed aqueous material I; and the remaining one (hereinafter called "processed aqueous material III") which is characterized in that it comprises an aqueous phase component produced at the time of the extraction of oils and fats from lamprey or a ground material of lamprey by boiling, or comprises a filtrate prepared by filtering the aqueous phase component.

The component having a peripheral vasoconstricting action, a peripheral vasodilating action, a myocardial constractile force increasing action, a spontaneous motion stimulating action, etc. can be taken in more efficiently by drinking or eating the processed solid material and/or the processed aqueous material of the present invention compared with the case where lamprey is cooked to be eaten as it is. Further, the processed aqueous material of lamprey according to the present invention can be used as a raw material for production of medicines.

EP 0 438 596 A1

## BEST MODES OF EMBODIMENTS OF THE INVENTION

First, the processed solid material of lamprey according to the present invention will be described hereinbelow. The processed solid material comprises a residue prepared by at least once performing an oil and fat extracting operation on lamprey or its ground material, or comprises a dried material of the residue. The term "lamprey" used herein means not only an individual of a lamprey but a part of the individual, for example, single materials such as muscular tissue, skin tissue, internal crgans, a head, eggs, etc. and combinations of the single materials. The term "ground material of lamprey" used herein means a material prepared by grinding lamprey as a raw material by means of a mixer, a chopper, etc.

As an operating technique for extracting oils and fats from lamprey or its ground material, an extracting method used for obtaining fish oil can be used as it is. In particular, it is preferable to use a solid-liquid extracting operation using an organic solvent as an extractant or an extracting operation using a supercritical fluid as an extractant.

The solid-liquid extracting operation using an organic solvent as an extractant can be conducted by an ordinary technique of stirring and extraction with a single organic solvent such as acetone, ethyl ether, ethyl alcohol, isopropyl alcohol, hexane, etc. or a mixture thereof as an extractant. Though the solid-liquid extracting operation may be conducted only once, it is preferable that the operation is repeated with one organic solvent or different organic solvents, for the purpose of sufficiently obtaining (removing) oils and fats contained in lamprey or its ground material. The processed aqueous material I, which will be described later, can be produced easily by repeating the solid-liquid extracting operation using one organic solvent or different organic solvents to sufficiently remove oils and fats contained in lamprey or its ground material.

On the other hand, the extracting operation using a supercritical fluid as an extractant can be conducted by a method in which an extractant (supercritical fluid) is recovered after lamprey or its ground material has been brought into contact, in a closed system, with $CO_2$ which exceeds critical temperature and critical pressure as the supercritical fluid; the extractant is returned to ordinary temperature and ordinary pressure after the contact; and oils and fats are recovered.

The processed solid material of lamprey according to the present invention comprises a residue prepared after at least once performing the aforementioned extracting operation on lamprey or its ground material, or comprises a dried material of the residue. The treatment in the case of providing the processed material in a dry form is not specifically limited. For example, a drying treatment under reduced pressure, an air-drying treatment, a heat-drying treatment, etc. can be used. Because the dried material is suitable for preservation, transportation, etc., it is preferable that the drying treatment is made sufficiently in the case where the processed material is provided as a dried material.

In the case where a processed solid material (dried material) of lamprey of the present invention is prepared by application of the solid-liquid extracting operation using an organic solvent as an extractant, a processed solid material (dried material) with very low content in water can be produced by further performing a solid-liquid extracting operation using ethyl ether or the like as an extractant on (i) a residue at the last stage of the solid-liquid extracting operation, or (ii) a dried material prepared by once drying the residue, and repeatedly applying a drying treatment to the residue if necessary.

In the case where a processed solid material (dried material) of lamprey according to the present invention is produced by performing an extracting operation using a supercritical fluid as an extractant, a processed solid material (dried material) of lamprey with very low content in water can be produced by applying the solid-liquid extracting operation using ethyl ether or the like as an extractant to a dried material prepared by once drying the residue, and repeatedly applying the drying treatment to the residue if necessary.

The processed solid material of lamprey of the present invention thus prepared contains a component having a peripheral vasoconstricting acticn, a peripheral vasodilating action, a myocardial constractile force increasing action, a spontaneous motion stimulating action, etc. Accordingly, prevention/therapy of circulatory disease and promotion of health can be attained more efficiently by eating the processed solid material as it is or by eating food to which the processed solid material was added (said processed solid material or said food may be cooked if necessary), compared with the case where non-processed lamprey is cooked and eaten as it is. Further, the processed solid material of lamprey of the present invention may be used as a raw material for production of the processed aqueous material I of lamprey which will be described later.

In the case where the processed solid material of lamprey is eaten for the purpose of an immediate efficacy for prevention/ therapy of circulatory disease, in particular, for therapy of the disease, it is preferable that it is eaten after eliminating only the peripheral vasoconstricting action. To eliminate only the peripheral vasoconstricting action, the processed solid material is treated with heat at a temperature of 50 to

3

EP 0 438 596 A1

100 °C for a period of 1 minute to 2 hours. By applying the heat treatment to the processed solid material, only the peripheral vasoconstricting action can be eliminated without eliminating the peripheral vasodilating action, myocardial constractile force increasing action, spontaneous motion stimulating action, etc.

Next, the processed aqueous material I of lamprey according to the present invention will be described hereinbelow. The processed aqueous material I comprises an extract solution produced by performing a solid-liquid extracting operation using water as an extractant on the aforementioned processed solid material of lamprey as a raw material. Here, the extracting operation can be made by an ordinary technique in the same manner as in the oase of producing the processed solid material by the solid-liquid operation using organic solvent as an extractant, except that water is used as an extractant. The term "water" used herein means: water usable as drinking water, such as purified water, distilled water, deionized water, tap water, etc.; and an aqueous solution containing the aforementioned water as a solvent, such as a physiological saline, a Ringer's solution, etc.

The processed aqueous material I of lamprey of the present invention thus prepared contains a component having a peripheral vasoconstricting action, a peripheral vasodilating action, a myocardial constractile force increasing action, a spontaneous motion stimulating action, etc. Accordingly, prevention/ therapy of circulatory disease and promotion of health can be attained more efficiently by eating the processed aqueous material I as it is or by eating food to which the processed aqueous material I was added, after they were cooked if necessary, compared with the case where non-processed lamprey is cooked and taken in as it is.

In the case where the processed aqueous material of lamprey is used for the purpose of an immediate efficacy for prevention/ therapy of circulatory disease, in particular, for therapy of the disease, it is preferable to use the processed aqueous material II which will be described later.

Next, the processed aqueous material II of lamprey according to the present invention will be described hereinbelow. The heat-treated material is produced by applying a heat treatment at a liquid temperature of 50 to 100 °C for a period of 1 minute to 2 hours to the aforementioned processed aqueous material I.

If the liquid temperature for the heat treatment is lower than 50 °C, a material having a peripheral vasoconstricting action remains undesirably. If the liquid temperature is higher than 100 °C, the preferred physical activity such as a myocardial constractile force increasing action decreases undesirably. If the treatment time is shorter than 1 minute, a material having a peripheral vasoconstricting action remains undesirably. If the treatment time is longer than 2 hours, the preferred physical activity such as a myocardial constractile force increasing action decreases undesirably.

The processed aqueous material II of lamprey of the present invention thus prepared contains a component having a peripheral vasoconstricting action, a peripheral vasodilating action, a myocardial constractile force increasing action, a spontaneous motion stimulating action, etc., but the component having the peripheral vasoconstricting action is inactivated. Accordingly, prevention/therapy of circulatory disease and promotion of health can be attained more efficiently by eating the processed aqueous material II as it is or by eating food to which the processed aqueous material II was added, after they are cooked if necessary, compared with the case where non-processed lamprey is cooked and eaten as it is. In particular, the immediate efficacy for therapy of circulatory disease can be improved. The processed aqueous material II can be used as a raw material for production of medicines in the same manner as the processed aqueous material I.

Next, the processed aqueous material III of lamprey according to the present invention will be described hereinbelow. The processed aqueous material III comprises an aqueous phase component produced at the time of obtaining oils and fats from lamprey or its ground material by a boiling technique or comprises a filtrate prepared by filtering the aqueous phase component.

The term "boiling technique" used herein means a wet extracting technique used for obtaining fish oil. In general, to obtain fish oil by the boiling technique is made by heating a raw material together with hot water, hot saline, dilute alkaline, water vapor, etc. to destroy a cell membrane of the raw material and elute an oil out of the cell membrane, and then separating an oil phase through a centrifugal separator or collecting an oil phase floated by leaving it still.

The processed aqueous material III of lamprey according to the present invention comprises an aqueous phase component prepared after separating the oil phase through centrifugal separation or after obtaining oil floated by leaving it still, in the aforementioned boiling technique, or comprises a filtrate prepared by filtering the aqueous phase component. It is a material of course that the solvent used when obtaining oils and fats from lamprey by the boiling technique is not specifically limited and that hot water, hot saline, dilute alkaline, water vapor, etc. can be used as described above. In particular, the preferred solvent is water vapor.

For example, the operation in the case where the processed aqueous material of lamprey of the present

4

invention is produced by the boiling technique using water vapor as a solvent is as follows.

First, lamprey or its ground material is put into a pressure vessel and boiled for a period of 0.1 to 2 hours while the inner pressure of the pressure vessel is set in a range of 120 to 300 kPa and while high-pressure water vapor at about 150 °C is blown into the pressure vessel. Then, the contents in the pressure vessel after boiling is centrifuged at about 2000 rpm to separate a micells portion (hereinafter called "micells i") and a mince portion. Then, the mince portion thus prepared is pressed by a technique such as a screw press technique, etc. to separate a micells portion (hereinafter called "micells ii") and a solid-phase component. Then, the micells i and/or the micells ii was centrifuged at 6000 - 20000 rpm to separate an aqueous phase component and an oil phase component. Thus, the aqueous phase component is obtained.

Also, the processed aqueous material III of lamprey of the present invention thus prepared contains a component having a peripheral vasoconstricting action, a peripheral vasodilating action, a myocardial constractile force increasing action, a spontaneous motion stimulating action, etc., but the component having the peripheral vasoconstricting action is inactivated. Further, the processed aqueous material III of lamprey of the present invention can be used as a raw material for production of medicines in the same manner as the processed aqueous materials I and II.

In the case where the processed aqueous materials I, II and III of lamprey according to the present invention are used as raw materials for production of medicines, an excipient such as glucose, milk sugar, etc. may be added to the processed aqueous materials for the production of an oral medicine, or an ointment such as vaseline, etc. may be also added thereto or an adequate absorption agent may be used for the production of a transdermal medicine.

In the aforementioned processed solid material and the processed aqueous material of lamprey according to the present invention, the component of lamprey having an immediate efficacy for prevention/therapy of circulatory disease, in particular, for therapy of the disease, can be taken in, in particular, in the case where eggs of lamprey are used as a raw material for lamprey or its ground material.

The present invention will be described more in detail with reference to the following examples.

Example 1

A processed solid material of lamprey according to the present invention was produced as follows.

First, the following materials (1) through (6) were prepared as crude materials for ground materials of lamprey.

(1) Lamprey [river lamprey (*Lampetra appendix*), the same shall apply hereinafter] after removing its head, liver and skin tissue;
(2) Egg of lamprey;
(3) Liver of lamprey;
(4) Skin tissue of lamprey;
(5) Head of lamprey; and
(6) Lamprey cut into irregular lumps after removing its head.

Then, ground materials were respectively prepared by grinding the crude materials (1) through (6) by using a mixer.

Then, a solid-liquid extracting operation using acetone as an extractant was applied twice to each of the ground materials thus prepared. The respective operation was carried out by adding 1 ℓ of acetone to 1 kg of each ground material, stirring the resulting mixture for 15 minutes and then filtering it.

After the second solid-liquid extracting operation, a processed solid material (residue) of lamprey prepared by filtration was evaporated in vacuo and dried to prepare a processed solid material (dried material) of lamprey.

Thereafter, the processed solid materials (dried materials) prepared from the crude materials (1), (2), (3) and (6) were finely pulverized by using a mortar and then were passed through a sieve to remove unpulverized materials shaped like strings and/or blocks. Then, an extracting operation with stirring for 15 minutes was performed on the resulting powder by using the same amount of ethyl ether as an extractant to thereby prepare processed solid materials (dried materials) (11), (12), (13) and (16) of lamprey with low content in water. Because the processed solid materials (dried materials) of lamprey prepared from the crude materials (4) and (5) could not pulverized in a mortar, the same ethyl ether extracting operation as in the cases of the processed solid materials (dried materials) (11), (12), (13) and (16) prepared from the crude materials (1), (2), (3) and (6) was made by using the processed solid materials (dried materials) as they were to thereby prepare processed solid materials (dried materials) (14) and (15) of lamprey with low content in water.

The crude materials used for preparation of processed solid materials (dried materials) of lamprey with low content in water, the weights thereof, the weights of processed solid materials (dried materials) produced in the process of preparation of processed solid materials (dried materials) of lamprey with low content in water, and the weights and yields of processed solid materials of lamprey of low water content as final products were shown in Table 1.

TABLE 1

| No. | Raw material used | | Total weight of the dried material*1 [g] | Processed solid material with low content in water (dried material) | | |
|---|---|---|---|---|---|---|
| | Kind | Weight[g] | | No. | Weight[g] | Yield [%] |
| (1) | Lamprey after removing its head, internal organs and cutaneous tissue | 3920 | 258.2 | (11) | 200 | 5.1 |
| (2) | Egg of lamprey | 300 | 72.3 | (12) | 34.3 | 11.4 |
| (3) | Liver of lamprey | 70 | 11.8 | (13) | 4.7 | 6.7 |
| (4) | Skin tissue of lamprey | 500 | 130 | (14) | 130*2 | 26.0 |
| (5) | Head of lamprey | 1050 | 280 | (15) | 280*2 | 26.7 |
| (6) | Lamprey cut into irregular lumps after removing its head | 3980 | 306.6 | (16) | 230 | 5.1 |

*1 : This means a processed solid material (dried material) produced in the process of preparation of a low-water-content processed solid material (dried material).

*2 : It remained still shaped like strings or blocks and could not be ground into powder in a mortar.

Example 2

Into a predetermined amount of each of the processed solid materials (dried materials) (11) - (16) of lamprey of low water content produced in Example 1, 10 ml of a physiological saline per 1 g of each processed solid material was added as an extractant. An extracting operation with stirring for an hour was performed on the resulting mixture. Then, the extract was filtered by using an aspirator to thereby prepare processed aqueous materials I (21) - (26) of lamprey.

Example 3

The processed aqueous materials I (21) - (26) of lamprey prepared in Example 2 were respectively heated to boil for 10 minutes and then filtered to thereby prepare processed aqueous materials III (31) - (36) of lamprey.

Example 4

A processed aqueous material III of lamprey was prepared as follows, by boiling using water vapor as a solvent.

First, 1 kg of lamprey was ground by a chopper to prepare a ground material of lamprey. The ground material was put into a pressure vessel and boiled for 2 hours while the inner pressure of the pressure vessel was set to be 300 kPa and, at the same time, high-pressure water vapor at 150 °C was blown into the pressure vessel. Then, the content of the pressure vessel after boiling was centrifuged at 2000 rpm to separate a micells portion (micells i) and a mince portion. Then, the mince portion thus prepared was pressed by a screw press to separate a micells portion (micells ii) and a solid-phase component. Then, the mixture of the micells i and the micells ii was centrifuged at 20000 rpm to separate an aqueous phase component and an oil phase component. The aqueous phase component thus prepared was filtered to prepare a filtrate, that is, a processed aqueous material III (filtrate) (41).

Efficacy test example 1

The following test was made to examine whether the processed aqueous materials I (21) - (26) of lamprey prepared in Example 2, the processed aqueous materials II (31) - (36) of lamprey prepared in Example 3 and the processed aqueous material III (41) of lamprey prepared in Example 4 have a peripheral vasodilating action.

After administeration of the processed aqueous materials I (21) - (26), the processed aqueous materials II (31) - (36) or the processed aqueous material III (filtrate) (41) of lamprey into the femoral artery of a dog anesthetized with an aqueous solution of sodium pentobarbital at 30 mg/kg intravenously, the change of the blood flow was measured with an electromagnetic flowmeter and then converted into the blood flow in the case where papaverine hydrochloride was administered to thereby examine whether a peripheral vasodilating action was exhibited.

Results of the examination are shown in Table 2.

7

TABLE 2

| Tested solution[1] | | | Vasodilating action[5] (dosage calculated in the term of papaverine hydrochloride) |
|---|---|---|---|
| Material | | No. | |
| Processed aqueous material I[2] | | (21) | 300 ± 58 |
| | | (22) | 320 ± 76 |
| | | (23) | 297 ± 41 |
| | | (24) | 250 ± 38 |
| | | (25) | 224 ± 44 |
| | | (26) | 234 ± 45 |
| Processed aqueous material II[3] | | (31) | 396 ± 43 |
| | | (32) | 480 ± 69 |
| | | (33) | 416 ± 83 |
| | | (34) | 352 ± 53 |
| | | (35) | 330 ± 62 |
| | | (36) | 550 ± 44 |
| Processed aqueous material III[4] | | (41) | 378 ± 56 |

*1 :   The dosage of each tested solution was 0.1 to 0.3 ml.
*2 :   The aqueous extracts I (21) - (26) were prepared from the low-water-content processed solid materials (dried materials) (11) -(16) prepared from the following crude materials (1) - (6), respectively.
   (1) 3920 g of lamprey after removing its head, internal organs and skin tissue.
   (2) 300 g of egg of lamprey.
   (3) 70 g of liver of lamprey.
   (4) 500 g of head of lamprey.
   (5) 1050 g of skin tissue of lamprey.
   (6) 3980 g of chopped lamprey after removing its head.
*3 :   The processed aqueous materials II (31) - (36) were prepared by performing a heat treatment on the aqueous extracts I (21) - (26).
*4 :   The processed aqueous material III (41) was a filtrate prepared by filtering an aqueous phase component produced at the time of obtaining oils and fats from lamprey by a boiling technique using water vapor as a solvent.
*5 :   Each value represents (the mean) ± (standard error). The unit thereof is [ $\mu$ g/ml]. In the respective case, the number of samples is 3.

As was obvious from Table 2, a peripheral vasodilating action was exhibited, in the case of administering the processed aqueous materials I (21) - (26) of lamprey prepared in Example 2, in the case of administering the processed aqueous materials II (31) - (36) of lamprey prepared in Example 3, or in the case of administering the processed aqueous material III (filtrate) (41) of lamprey prepared in Example 4. In particular, it was found that an excellent peripheral vasodilating action was exhibited in the case of administering the processed aqueous materials II (31)~(37) of lamprey prepared in Example 3 and the processed aqueous material III (filtrate) (41) of lamprey prepared in

Example 4.

Further, it was found that a processed aqueous material II containing a substance having peripheral vasodilating action was prepared most efficiently in the case where egg of lamprey was used as a crude material, judging from the amount of the crude material used for production of the processed aqueous materials.

Efficacy test example 2

The following test was made to examine whether the processed aqueous materials I (21) - (26) of lamprey prepared in Example 2, the processed aqueous materials II (31) - (36) of lamprey prepared in Example 3 and the processed aqueous material III (41) of lamprey prepared in Example 4 have a myocardial contractile force increasing action or not.

The right atrium removed from a guinea pig so as to contain the sinus node was suspended in an organ bath filled with Tyrode solution at 37°C which was bubbled with a gas mixture of 95 % $O_2$ and 5 % $CO_2$. The processed aqueous materials I (21) - (26), the processed aqueous materials II (31) - (36) and the processed aqueous material III (41) of lamprey were respectively applied, in the amount of 0.06 ml, to the organ bath. After 5 minutes, the myocardial contractile force and the heart rate were measured. The myocardial contractile force and the heart rate were compared with those obtained before the addition of the processed aqueous materials I (21) - (26), the processed aqueous materials II (31) - (36) or the processed aqueous material III (41) of lamprey, to calculate the change rates thereof.

As a comparison, a physiological saline was used instead of the processed aqueous materials I (21) - (26), the processed aqueous materials II (31) - (36) and the processed aqueous material III (41). The myocardial contractile force and the heart rate after 5 minutes from the application of the physiological saline were compared with those obtained before the application of the physiological saline in the same manner as described above, to calculate the change rates thereof.

The change rates were compared with each other to examine whether a myocardial contractile force increasing action was exhibited in the case of the application of the processed aqueous materials I (21) - (26), the processed aqueous materials II (31) - (36) or the processed aqueous material III (41) of lamprey.

Results of the examination are shown in Table 3.

EP 0 438 596 A1

TABLE 3

| Tested solution[1] | | Change rate[5]   [%] | |
| Material | No. | Myocardial contractile force | Heart rate |
|---|---|---|---|
| Processed aqueous material I[2] | (21) | 37.5 ± 26.1 | 0.3 ± 1.4 |
| | (22) | 26.8 ± 9.3 | 6.3 ± 2.6 |
| | (23) | 29.0 ± 12.0 | 5.0 ± 1.8 |
| | (24) | 18.2 ± 6.3 | 1.8 ± 2.0 |
| | (25) | 21.3 ± 3.0 | 4.2 ± 1.7 |
| | (26) | 24.5 ± 11.0 | 7.3 ± 3.6 |
| Processed aqueous material II[3] | (31) | 21.2 ± 11.3 | 6.3 ± 5.5 |
| | (32) | 31.3 ± 14.4 | 5.0 ± 5.5 |
| | (33) | 30.0 ± 13.0 | 1.8 ± 1.2 |
| | (34) | 33.0 ± 14.0 | 6.3 ± 2.5 |
| | (35) | 16.8 ± 7.8 | 5.2 ± 1.3 |
| | (36) | 19.3 ± 6.5 | 3.2 ± 1.5 |
| Processed aqueous material III[4] | (41) | 15.3 ± 5.7 | 5.5 ± 1.8 |
| Physiological saline | | 0.2 ± 4.3 | 2.2 ± 0.6 |

*1 :    The dosage of each tested solution was 0.06 ml.
*2 :    The aqueous extracts I (21) - (26) were prepared from
the low-water-content processed solid materials (dried
materials) (11) -(16) prepared from the following crude
materials (1) - (6), respectively.
(1) 3920 g of lamprey after removing its head, internal
    organs and skin tissue.
(2) 300 g of egg of lamprey.
(3) 70 g of liver of lamprey.
(4) 500 g of head of lamprey.
(5) 1050 g of skin tissue of lamprey.
(6) 3980 g of chopped lamprey after removing its head.
*3 :    The processed aqueous materials II (31) - (36) were
prepared by applying a heat treatment to the aqueous
extracts I (21) - (26).
*4 :    The processed aqueous material III (41) was a filtrate
prepared by filtering an aqueous phase component produced
at the time of obtaining oils and fats from lamprey by
a boiling technique using water vapor as a solvent.

10

*5 :     The change rate represents [(the mean) ± (standard error)] at 5 minutes after the addition of the tested solution against a value before the addition. In the cases of the processed aqueous materials I, II and III, the number of samples is 4. In the case of the physiological saline, the number of samples is 3.

As was obvious from Table 3, a myocardial contractile force increasing action and an increase of the heart rate were exhibited, in the case of application of the processed aqueous materials I (21) - (26) of lamprey prepared in Example 2, in the case of application of the processed aqueous materials II (31) - (36) of lamprey prepared in Example 3, or in the case of application of the processed aqueous material III (filtrate) (41) of lamprey prepared in Example 4. In particular, it was found that the increasing rate of the myocardial contractile force was larger than the increasing rate of the heart rate.

Efficacy test example 3

The following test was made to examine whether the low-water-content processed solid materials (dried materials) (11) - (16) of lamprey prepared in Example 1, the processed aqueous materials I (21) - (26) of lamprey prepared in Example 2, the processed aqueous materials II (31) - (36) of lamprey prepared in Example 3 and the processed aqueous material III (41) of lamprey prepared in Example 4 have an action of stimulating the quantity of motion.

After daily oral administration, 40 mg per day, of the processed solid materials (dried materials) (11) - (16) of lamprey or 0.5 ml per day of the processed aqueous materials I (21) - (26), the processed aqueous materials II (31) - (36) and the processed aqueous material III (filtrate) (41) of lamprey to mice over 14 days, the mice were placed in wheel cages to measure the number of the revolutions of the cages for 30 minutes.

For reference, mice after daily oral administration of physiological saline for 14 days were placed in wheel cages to measure the number of revolutions of the cages for 30 minutes.

The numbers of the revolutions were compared with each other to examine whether an action of stimulating the motion was exhibited in the cases of oral administration of the processed solid materials (dried materials) (11) - (16), the processed aqueous materials I (21) - (26), the processed aqueous materials II (31) - (36) and the processed aqueous material III (filtrate) (41) of lamprey.

Results of the examination are shown in Table 4.

TABLE 4

| Tested solution[1] | | | The number of the revolutions[6] in the wheel cage |
| | | | |
| Material | | No. | (30-minutes counts) |
|---|---|---|---|
| Processed solid material [2] (dried material) | | (11) | 592 ± 89 |
| | | (12) | 625 ± 83 |
| | | (13) | 562 ± 63 |
| | | (14) | 572 ± 73 |
| | | (15) | 558 ± 46 |
| | | (16) | 613 ± 81 |
| Processed aqueous material I[3] | | (21) | 647 ± 45 |
| | | (22) | 598 ± 65 |
| | | (23) | 546 ± 36 |
| | | (24) | 611 ± 48 |
| | | (25) | 585 ± 63 |
| | | (26) | 549 ± 43 |
| Processed aqueous material II[4] | | (31) | 640 ± 63 |
| | | (32) | 611 ± 45 |
| | | (33) | 586 ± 91 |
| | | (34) | 574 ± 68 |
| | | (35) | 536 ± 72 |
| | | (36) | 552 ± 78 |
| Processed aqueous material III[5] | | (41) | 583 ± 45 |
| Physiological saline | | | 508 ± 37 |

*1 :   The dosage of each of the processed solid materials (dried materials) (11) - (16) was 40 mg per day. The dosage of each of the other tested materials was 0.5 ml.

*2 :   The processed solid materials (dried materials) (11) - (16) were low-water content processed solid materials prepared from the following crude materials (1) - (6), respectively.
(1) 3920 g of lamprey after removing its head, internal

12

```
          organs and skin tissue.
    (2) 300 g of egg of lamprey.
    (3) 70 g of liver of lamprey.
    (4) 500 g of head of lamprey.
    (5) 1050 g of skin tissue of lamprey.
    (6) 3980 g of chopped lamprey after removing its head.
*3 :      The aqueous extracts I (21) - (26) were prepared from
     the low-water-content processed solid materials (dried
     materials) (11) -(16).
*4 :      The processed aqueous materials II (31) - (36) were
     prepared by applying a heat treatment to the aqueous
     extracts I (21) - (26).
*5 :      The processed aqueous material III (41) was a filtrate
     prepared by filtering an aqueous phase component produced
     at the time of obtaining oils and fats from lamprey by
     a boiling technique using water vapor as a solvent.
*6 :      Each value represents (the mean) ± (standard error).
     In the respective case, the number of samples is 10.
```

As was obvious from Table 4, it was found that an excellent effect of motor activity was exerted, in the case of repeated administration of the low-water content processed solid materials (dried materials) (11) - (16) prepared in Example 1, in the case of repeated administration of the processed aqueous materials I (21) - (26) of lamprey prepared in Example 2, in the case of repeatedly administrating the processed aqueous materials II (31) - (36) of lamprey prepared in Example 3, or in the case of repeated administration of the processed aqueous material III (filtrate) (41) of lamprey prepared in Example 4. In particular, it was found that the increase in motor activity lasted.

As described above, each of the processed solid materials and the processed aqueous materials I of lamprey according to the present invention contains a component having a peripheral vasoconstricting action, a peripheral vasodilating action, a myocardial contractile force increasing action, a spontaneous motion stimulating action, etc.

Accordingly, the aforementioned useful component contained in lamprey can be eaten more efficiently by the present invention compared with the case where lamprey is cooked and eaten as it is. Further, medicines and healthy foods having efficacy for prevention/therapy of circulatory disease and for promotion of health can be provided. In particular, medicines and healthy foods having an immediate effect on circulatory disease can be provided.

## Claims

1. A processed solid material of lamprey characterized in that said processed solid material comprises a residue prepared by at least once application of oils and fats extracting operation onto lamprey or its ground material, or comprises a dried material of said residue.

2. A processed solid material of lamprey according to Claim 1, in which said oils and fats extracting operation is a solid-liquid extracting operation using an organic solvent as an extractant or an extracting operation using a supercritical fluid as an extractant.

3. A processed aqueous material of lamprey, characterized in that said processed aqueous material comprises an extracted liquid prepared by applying a solid-liquid extracting operation using water as an extractant to a processed solid material of lamprey as defined in Claim 1 or 2.

4. A processed aqueous material of lamprey, characterized in that said processed aqueous material is prepared by applying a heat treatment at a liquid temperature of 50 to 100 °C for a period of 1 minute to 2 hours to a processed aqueous material of lamprey as defined in Claim 3.

5. A processed aqueous material of lamprey, characterized in that said processed aqueous material comprises an aqueous phase component produced at the time of obtaining oils and fats from lamprey by a boiling technique, or a filtrate prepared by filtering said aqueous phase component.

6. A processed aqueous material of lamprey according to Claim 5, in which said boiling technique uses water vapor as a solvent.

# INTERNATIONAL SEARCH REPORT

International Application No **PCT/JP90/00912**

## I. CLASSIFICATION OF SUBJECT MATTER (if several classification symbols apply, indicate all) ⁶

According to International Patent Classification (IPC) or to both National Classification and IPC

Int. Cl⁵  A61K35/60

## II. FIELDS SEARCHED

| Minimum Documentation Searched ⁷ | |
|---|---|
| Classification System | Classification Symbols |
| IPC | A61K35/60, A23L1/325, A23L1/30, A23B4/03 |

Documentation Searched other than Minimum Documentation
to the Extent that such Documents are Included in the Fields Searched ⁸

## III. DOCUMENTS CONSIDERED TO BE RELEVANT ⁹

| Category * | Citation of Document, ¹¹ with indication, where appropriate, of the relevant passages ¹² | Relevant to Claim No. ¹³ |
|---|---|---|
| A | JP, A, 49-108263 (Ajinomoto Co., Inc.), 15 October 1974 (15. 10. 74), Claim (Family: none) | 1 - 6 |
| A | JP, A, 57-181654 (Shuzo Nakazono), 9 November 1982 (09. 11. 82), Claim (Family: none) | 1 - 6 |
| A | JP, B1, 47-46322 (Toyo Jozo Co., Ltd.), 22 November 1972 (22. 11. 72), Claim & US, A, 3822348 | 1 - 6 |

\* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| September 17, 1990 (17. 09. 90) | October 1, 1990 (01. 10. 90) |
| International Searching Authority | Signature of Authorized Officer |
| Japanese Patent Office | |

Form PCT/ISA/210 (second sheet) (January 1985)